# EUROPEAN PATENT SPECIFICATION

(11) **EP 2 011 430 B1**
(45) Date of publication and mention of the grant of the patent: **09.11.2011**
(21) Application number: 07742397.8
(22) Date of filing: 25.04.2007
(51) Int. Cl.: A61B 1/00

(54) **ANTENNA UNIT AND RECEIVING SYSTEM**
ANTENNENEINHEIT UND EMPFANGSSYSTEM
SYSTEME DE RECEPTION ET UNITE D'ANTENNE

(30) Priority: 26.04.2006 JP 2006122683
(43) Date of publication of application: 07.01.2009
(73) Proprietor: Olympus Medical Systems Corp., Tokyo 151-0072 (JP)
(72) Inventor: SHIGEMORI, Toshiaki, Tokyo 151-0072 (JP)
(74) Representative: von Hellfeld, Axel
(86) International application number: PCT/JP2007/058962
(87) International publication number: WO 2007/125962

(56) References cited:
- EP-A- 1 329 189
- WO-A-2006/028134
- JP-A- 2001 231 186
- JP-A- 2006 075 244

## Description

### TECHNICAL FIELD

The present invention relates to an antenna unit which receives a radio signal transmitted from a body-insertable device, and a receiving system.

### BACKGROUND ART

In recent years, a capsule endoscope including an imaging function and a radio communication function has appeared in the field of an endoscope. The capsule endoscope has functions of traveling, after it is swallowed from a mouth of an examinee as a subject for an observation (examination), inside of organs (inside of a body cavity) such as the stomach and the small intestine according to their peristalsis and sequentially capturing their images by using the imaging function during an observation period until it is naturally excreted from a living body of the subject.

Image information captured in the body cavity by the capsule endoscope, during the observation period when the capsule endoscope travels inside the organs, is sequentially transmitted to an outside of the subject body through the radio communication function, received by an external receiving system, and thereby stored in a memory provided in the external receiving system. By carrying the receiving system having the radio communication function and the memory function, the examinee can move freely without feeling inconveniences even during the observation period which starts from the swallow of the capsule endoscope and ends by the excretion of the capsule endoscope. After the observation by using the capsule endoscope, doctors or nurses make a display unit such as a screen display device show the image information of the inside of the body cavity stored in the memory of the receiving system so that they can make a diagnosis (see Patent Document 1).

Patent Document 1: Japanese Patent Application Laid-Open No. 2001-231186 (page 3, FIG. 1)

### DISCLOSURE OF INVENTION

### PROBLEM TO BE SOLVED BY THE INVENTION

Incidentally, the conventional capsule endoscope system includes an antenna unit which has a plurality of antennas that receives a radio signal transmitted from the capsule endoscope, and an external unit which processes an intermediate signal, a base band signal, a radio frequency signal in some cases, and the like received by the antenna unit to generate and store image information, the antenna unit and the external unit being connected with a connector or with a cable via the connector. Here, since a connecting function of the connector significantly deteriorates due to an insertion and an ejection between the antenna unit and the external unit, the connector generally has a life shorter than other parts and the life becomes still shorter especially when it comes to a high frequency connector which transmits high frequency signal in particular. As a result of this, there are problems that a general life of a receiving system constituted by connecting the antenna unit and the external unit via a connector becomes shorter because of the connector, the general life of the receiving system cannot be extended, and a long-life receiving system cannot be realized.

In addition, in the receiving system constituted by connecting the antenna unit and the external unit via a connector, there are problems that a wiring work becomes complicated and a patient physically suffers a great strain since it is necessary to connect the antenna unit and the external unit with the connector when the receiving system is attached to a patient as a subject by arranging the receiving antennas at desired positions on an outer surface of the subject.

Furthermore, when the antenna unit and the external unit are connected with the connector in the context that the receiving system including the receiving antennas is a medical device to which a sterilizing work is frequently performed by using an alcohol and the like, there are problems that a cap, which covers the connector part for securing a drop-proof property in sterilization, is of a bad operability, easily damaged, and of a possibility of being misusing.

The present invention is achieved in view of the weak points in the foregoing conventional technique, and an object of the present invention is to provide an antenna unit which realizes a long-life system, reduces a physical strain caused by a wiring work of a receiving system in attaching a receiving antenna to a subject body, and easily secures a drop-proof property of the receiving system; and to provide the receiving system.

### MEANS FOR SOLVING PROBLEM

WO 2006-028-34 discloses an antenna unit and a receiving apparatus linked either by a connector a by photocoupling.

A receiving system according to the present invention includes: an antenna unit including a first antenna which is arranged on an outer surface of a subject and receives a radio signal transmitted at a first frequency from a body-insertable device inserted into an inside of the subject, a main body part which is attached to an outside of the subject and includes a frequency converter that converts the signal of the first frequency received by the first receiving antenna into a signal of a second frequency and a transmitting antenna that transmits the signal of the second frequency as a radio signal, and a signal line which connects the first receiving antenna and
the main body part; and an external unit including a second receiving antenna which receives the radio signal of the second frequency transmitted from the transmitting antenna, and a reception processor which demodulates the radio signal received by the second receiving antenna to acquire information transmitted from the body-insertable device.

in the receiving system according to the present invention, the first receiving antenna and the signal line are provided in plural number, and the main body part includes received-strength detector that detects a received strength of the radio signal received by each of the first receiving antennas, a selection controller that selects one first receiving antenna for receiving the radio signal based on the received strength detected by the received-strength detector, and a switching unit that switches over to the first receiving antenna, selected by the selection controller, among the plurality of first receiving antennas.

In the receiving system according to the present invention, the maim body part further includes a converting unit that converts selected antenna identifying information for identifying the first receiving antenna selected by the selection controller into the second frequency, and performs one of inserting into and superimposing on the radio signal which is transmitted from the body-insertabie device and converted by the frequency converter, or
that converts, into the second frequency, antenna identifying information for identifying each of the first receiving antennas and the received strength of each radio signal received by each of the first receiving antennas which are detected by the received-strength detector, and
performs one of inserting into and superimposing on the radio signal which is transmitted from the body-insertable device and converted by the frequency converter.

### EFFECT OF THE INVENTION

In a receiving system according to the present invention, a radio signal transmitted at a first frequency from a body-insertable device inserted into:an inside of a body of a subject is received by a receiving antenna arranged on an outer surface of the subject, the signal of the first frequency input via a signal line is converted into a signal of a second frequency by a frequency converter, and the converted signal of the second frequency is transmitted as a radio signal via transmitting antenna. Accordingly, the antenna unit and an external unit are connected wirelessly and the connection between the antenna unit and the external unit does not require a connector whose life is generally short. As a result, the present invention has advantages that it is possible to extend a useful life of an entirety of the receiving system, to realize a long-life of the same, to reduce a physical strain on a. patient as the subject without a necessity of performing a complicated work of a cable wiring for connecting the antenna unit and the external unit on the body of the subject, and to easily secure a. drop-proof property due to no necessity of using the connector.

### BRIEF DESCRIPTION OF DRAWINGS

FIG. 1 is a view showing an entire structure of an intra-subject information acquiring system according to a first embodiment not falling under the scope of the claims;
FIG. 2 is a block diagram of a structure of a receiving system shown in FIG. 1;
FIG. 3 is a perspective view of an antenna unit shown in FIG. 2;
FIG. 4 is a perspective view showing an assembling of the antenna unit shown in FIG. 2;
FIG. 5 is a view showing a sterilizing operation of the antenna unit shown in FIG. 2;
FIG. 6 is a view showing an entire structure of an intra-subject information acquiring system according to a second embodiment;
FIG. 7 is a block diagram of a structure of a receiving system shown in FIG. 6;
FIG. 8 is a view showing a signal component output from an insertion unit shown in FIG. 7; and
FIG. 9 is a view showing another example of a signal component output from the insertion unit shown in FIG. 7.

### EXPLANATIONS OF LETTERS OR NUMERALS

- 1: Subject
- 2, 202: Receiving system
- 2a, 202a: Antenna unit
- 2b, 202b: External unit
- 3: Capsule endoscope
- 4: Display device
- 5: Portable recording medium
- 21a: Cable
- 22: Frequency converter
- 23: Transmitting antenna
- 24, 37: Power supply unit
- 24a: Battery housing part
- 24b: Battery
- 24c: Cover part
- 25: Sealing member
- 26a, 26b: Frame
- 27: Adhesive agent
- 28: Sterilizing solution
- 31, A1 to An: Receiving antenna
- 32: Receiver
- 33, 233: Siginal processor
- 34: Control unit
- 35: Storage unit
- 36: Display unit
- 241a: Hole part
- 241c: Protrusion part
- 221: Selecting unit
- 225: Intensity detector
- 226: Selection controller
- 227: Converter
- 228: Insertion unit

### BEST MODE(S) FOR CARRYING OUT THE INVENTION

Best modes (hereinafter simply referred to as "exemplary embodiments") of an antenna unit and a receiving system according to the present invention will be explained below with reference to the accompanying drawings. It should be noted that since the drawings are only schematic, a relation between a thickness and a width of each part, a ratio of a thickness of each part, and the like are different from those in the actual size, and as a matter of course that the drawings include a part having a different dimensional relation and a different ratio among the drawings. The same part is assigned with the same symbol in the description throughout the drawings. Though the embodiments will be explained below by taking the receiving system which, including the antenna unit, is applied to an intra-subject information acquiring system as one example, it is needless to say that an application field of the antenna unit and the receiving system is not necessarily limited to the intra-subject information acquiring system.

### First embodiment not falling under the scope of the claims

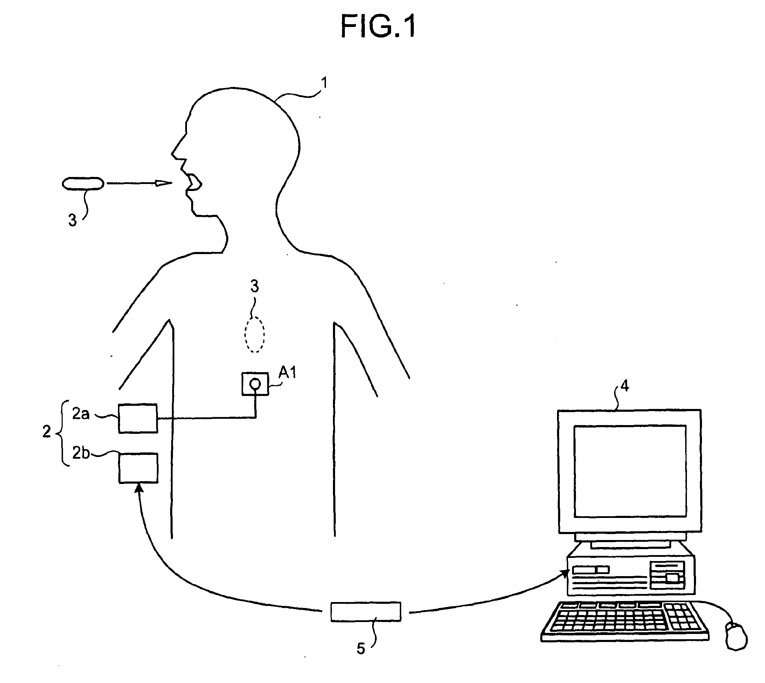

FIG. 1 is a view showing an entire structure of an intra-subject information acquiring system of a radio type according to a first embodiment not falling under the scope of the claims. In FIG. 1, the intra-subject information acquiring system includes a receiving system 2 having a radio receiving function, and a capsule endoscope 3 which is inserted into an inside of a body of a subject 1 to capture images of the inside of a body cavity and transmit image information to the receiving system 2. The intra-subject information acquiring system further includes a display device 4 which displays images of the inside of the body cavity based on the information included in a radio signal received by the receiving system 2, and a portable recording medium 5 which transfers data between the receiving system 2 and the display device 4.

The receiving system 2 includes an antenna unit 2a which has a receiving antenna A1, and an external unit 2b which performs a processing of the radio signal received by the receiving antenna A1. By wirelessly connecting the antenna unit 2a and the external unit 2b, the receiving system 2 outputs the radio signal received from the capsule endoscope 3 by the antenna unit 2a to the external unit 2b. With this wireless connection, the antenna unit 2a and the external unit 2b are not connected to each other by using a connector or a cable via the connector.

The display device 4 is for displaying and processing the images of the inside of the body cavity captured by the capsule endoscope 3, and has a work station or the like for displaying and processing the images based on data obtained by the portable recording medium 5. The display device 4 may have a configuration such that the images are displayed directly on a CRT display, a liquid crystal display or the like, or a configuration such that the images are output to another medium such as a printer.

The portable recording medium 5 is detachably attached to the external unit 2b and the display device 4, and has a configuration such that when inserted to be attached to both of them, information can be input or output. Specifically, the portable recording medium 5 is inserted to be attached into the external unit 2b and records the image information transmitted from the capsule endoscope 3 therein while the capsule endoscope 3 is moving in the body cavity of the subject 1. After the capsule endoscope 3 is excreted from the subject 1, namely, after the imaging of the inside of the subject 1 is completed, the portable recording medium 5 is removed from the external unit 2b and inserted to be attached into the display device 4, and the display device 4 reads out the information recorded in the portable recording medium 5. For example, by using the portable recording medium 5 such as CompactFlash (registered trademark) for the information transfer between the external unit 2b and the display device 4, the subject 1 can move freely during the imaging of the inside of the body cavity unlike the case such that the external unit 2b and the display device 4 are connected with each other by a wire. Here, though the portable recording medium 5 is used for the information transfer between the external unit 2b and the display device 4, the present invention is not limited to this. For example, another recording device such as a hard disc drive built into the external unit 2b may be used, and the external unit 2b and the display device 4 may be connected with or without a wire for the information transfer therebetween.

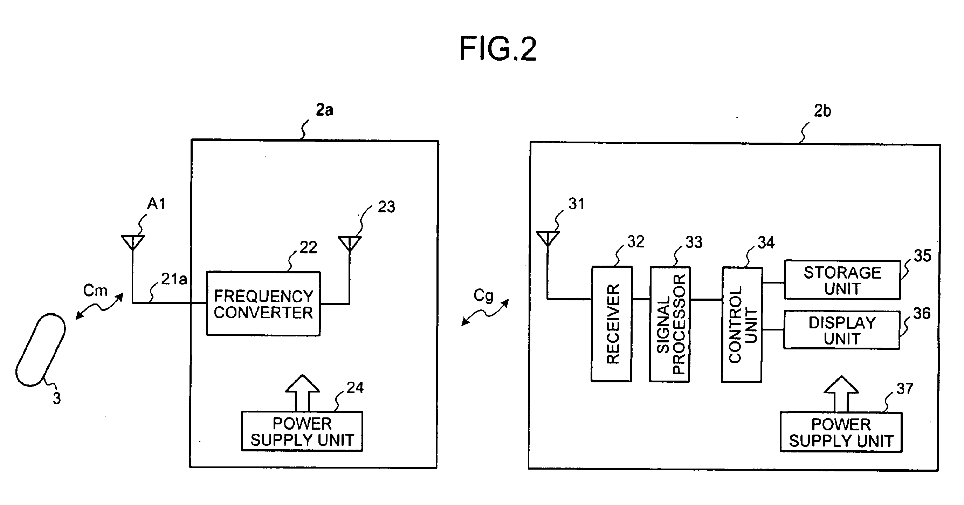

Next, the receiving system 2 will be explained. FIG. 2 is a block diagram schematically showing the entire structure of the receiving system 2. As shown in FIG. 2, the antenna unit 2a includes the receiving antenna A1, a frequency converter 22, a transmitting antenna 23, and a power supply unit 24. The receiving antenna A1 is for receiving a radio signal transmitted at a first frequency from the capsule endoscope 3. The receiving antenna A1 is realized by a loop antenna, which is fixed by sticking and the like at a desired position on an outer surface of the body of the subject 1, for example.

The frequency converter 22 converts the radio signal of the first frequency received via the receiving antenna A1 into a signal of a second frequency which is different from the first frequency. For example as shown in FIG. 2, when a radio signal is transmitted from the capsule endoscope 3 at a frequency of a megahertz range and the receiving antenna A1 receives the signal of the megahertz frequency, the frequency converter 22 performs upconverting in which the signal of the megahertz frequency received as the first frequency by the receiving antenna A1 is converted into a signal of a gigahertz range frequency which is the second frequency. The transmitting antenna 23 transmits the signal of the second frequency converted by the frequency converter 22. The power supply unit 24 supplies an electric power to each part in the antenna unit 2a.

On the other hand, the external unit 2b includes a receiving antenna 31, a receiver 32, a signal processor 33, a control unit 34, a storage unit 35, a display unit 36, and a power supply unit 37, as shown in FIG. 2. The receiving antenna 31 receives the radio signal of the second frequency transmitted from the antenna unit 2a. For example, the receiving antenna 31 has a function of receiving a gigahertz range frequency when a frequency of the radio signal transmitted from the transmitting antenna 23 is within the gigahertz range. The receiver 32 performs a reception processing such as a demodulation and an analogue/digital conversion with respect to the radio signal received via the receiving antenna 31. The signal processor 33 performs a processing of extracting image information by processing a digital signal output from the receiver 32. The control unit 34 performs a general control of the external unit 2b and an output control of information such as the image information input via the signal processor 33. The storage unit 35 stores the image information and the like captured by the capsule endoscope 3. The display unit 36 displays the image information and the like captured by the capsule endoscope 3. The power supply unit 37 supplies an electric power to each part, described above, of the external unit 2b.

Since the receiving system 2 according to the first embodiment is configured to wirelessly connect the antenna unit 2a and the external unit 2b and the antenna unit 2a and the external unit 2b are not connect by using a connector, a cable, or the like, it is possible to extend the useful life of the entirety of the receiving system 2 without using a connector whose life is generally short, and to extend the life of the entirety of the receiving system 2.

Besides, since the antenna unit 2a and the external unit 2b are not connected with a cable in the receiving system 2 according to the first embodiment, it is not necessary to perform a complicated work of a cable wiring for connecting the antenna unit 2a and the external unit 2b on the subject 1, and it is possible to reduce a physical strain on the patient as a subject.

Furthermore, by using a sealing member or an adhesive agent in performing a processing of attaching a battery constituting the power supply unit 24 and of assembling the frame, it is possible to easily secure a drop-proof property in the antenna unit 2a according to the first embodiment.

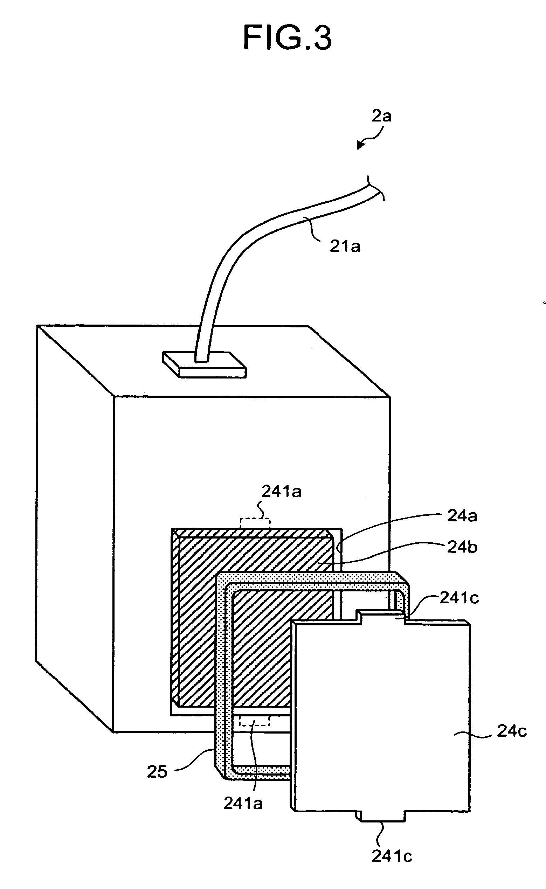

FIG. 3 is a perspective view showing the structure of the antenna unit 2a according to the first embodiment of the present invention. As shown in FIG. 3, a cable 21a, as a signal line electrically connecting a main body of the antenna unit 2a and the antenna A1 not shown, is extended from an upper surface of the antenna unit 2a. Further, the antenna unit 2a is provided with a battery housing part 24a which houses a battery 24b constituting the power supply unit 24 at its side surface. The battery housing part 24a after the battery 24b is attached therein is blocked by using a cover part 24c. The cover part 24c has a protrusion part 241c as shown,in FIG. 3 for example, and the battery housing part 24a has a hole part 241a which fits the protrusion part 241c. Furthermore, a sealing member 25 is provided between the cover part 24c and the battery housing part 24a, the sealing member 25 having an endless form in a size corresponding to an opening shape of the battery housing part 24a and being formed of a resin material having a stretching property.

In the antenna unit 2a, the protrusion part 241c of the cover part 24c is fitted in the hole part 241a of the battery housing part 24a to cover the battery housing part 24a with the cover part 24c in a state of arranging the sealing member 25 between the battery housing part 24a and the cover part 24c after the battery 24b is attached into the battery housing part 24a. The sealing member 25 arranged between the cover part 24c and the battery housing part 24a blocks an air gap between the cover part 24c and the battery housing part 24a due to a direct contact between the cover part 24c and the periphery of the battery housing part 24a, thereby preventing a liquid intrusion into the antenna unit 2a through the battery housing part 24a.

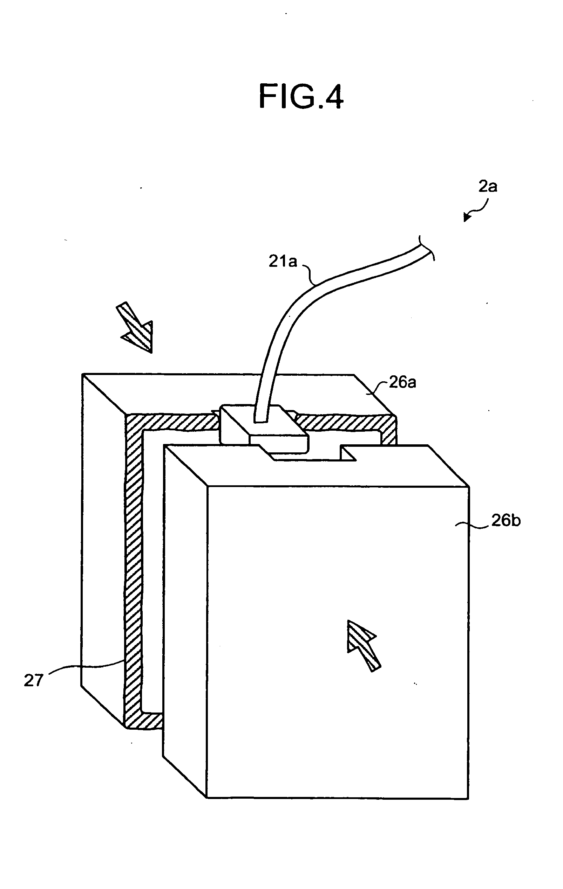

Moreover, when the antenna unit 2a is constituted by a combination of frames 26a and 26b as shown in FIG. 4, a drop-proof property is secured by using an adhesive agent 27 which is formed of a resin material and the like. For example, the antenna unit 2a is assembled by sticking the frame 26a and the frame 26b with an external force applied to the frames 26a and 26b respectively in directions shown by arrows in FIG. 4 after the adhesive agent 27 formed of a resin material and the like is applied to an adhesion part of at least one of the frames 26a and 26b. The adhesive agent 27 blocks an air gap between the frames 26a and 26b, thereby preventing a fluid intrusion into the antenna unit 2a through the air gap between the frames 26a and 26b.

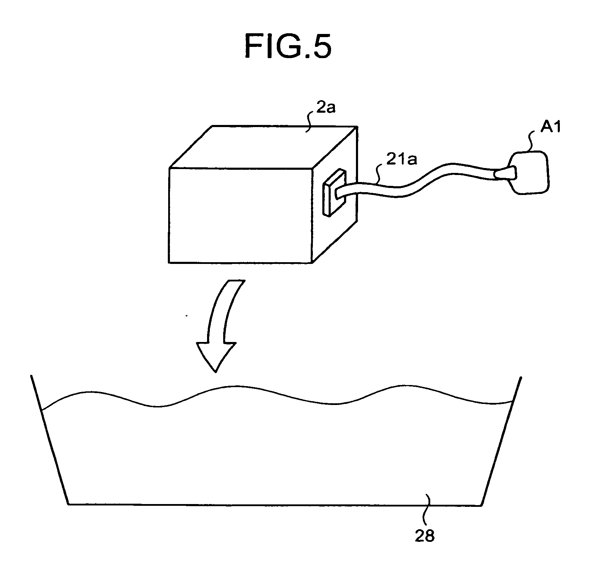

As described, since the antenna unit 2a according to the first embodiment is not provided with a connector and the like for an external connection, it is possible to easily assemble the antenna unit 2a by arranging a sealing member or an adhesive agent in an air gap part, and to secure a high drop-proof property. As a result of this, a sterilizing solution never intrudes into the inside of the antenna unit 2a even when a sterilizing operation for the antenna unit 2a is performed by soaking the antenna unit 2a directly into a sterilizing solution 28 as shown in FIG. 5, and thereby the sterilizing operation can be easily performed.

### Second embodiment

A second embodiment of the present invention will be explained next. In contrast to the first embodiment described above provided with one receiving antenna, a plurality of receiving antennas are provided in the second embodiment, and these receiving antennas are switched over to receive a radio signal including the image information captured by the capsule endoscope 3.

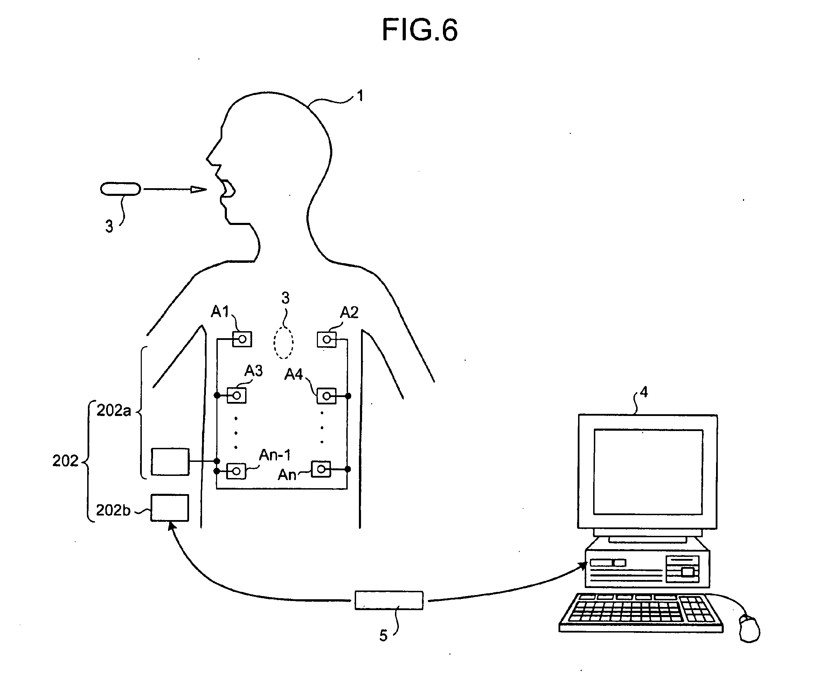

As shown in FIG. 6, a receiving system 202 according to the second embodiment includes an antenna unit 202a having a plurality of receiving antennas A1 to An, and an external unit 202b which processes a radio signal transmitted from the antenna unit 202a, the antenna unit 202a being provided instead of the antenna unit 2a according to the first embodiment and the external unit 202b being instead of the external unit 2b according to the first embodiment. The receiving antennas A1 to An may be attached on a surface of a clothing such as a jacket worn by the subject 1.

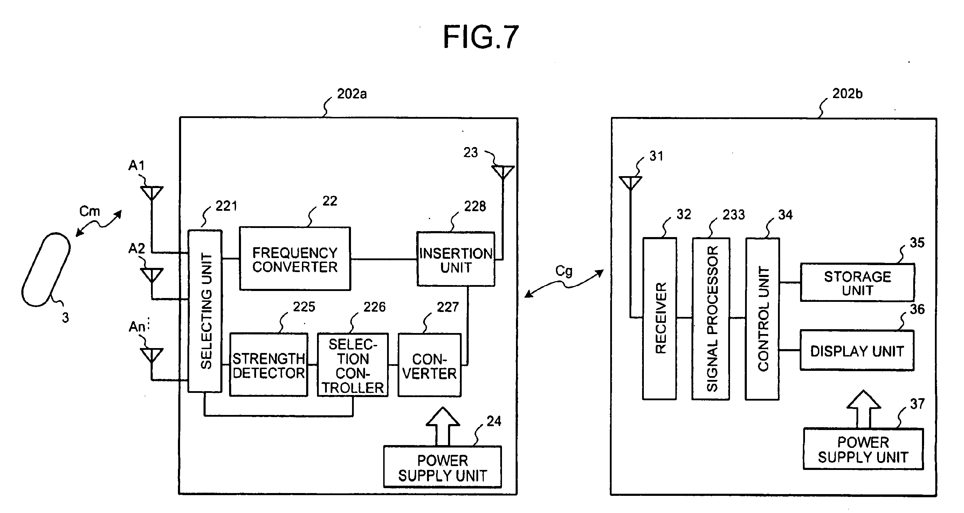

Next, the receiving system 202 will be explained with reference to FIG. 7. FIG. 7 is a schematic block diagram of an entire structure of the receiving system 202. As shown in FIG. 7, the antenna unit 202a has a configuration including the receiving antennas A1 to An, a selecting unit 221, an strength detector 225, a selection controller 226, a converter 227, and an insertion unit 228, compared to the antenna unit 2a according to the first embodiment.

The selecting unit 221, after outputting the radio signal received by each of the receiving antennas A1 to An to the strength detector 225, selects an antenna A which is suitable for receiving the radio signal among the receiving antennas A1 to An and outputs the radio signal received from the capsule endoscope 3 via the selected receiving antenna A to the frequency converter 22 under the control by the selection controller 226. The frequency converter 22 converts the radio signal which is transmitted from the capsule endoscope 3 and output from the selecting unit 221 from the first frequency to the second frequency which is different from the first frequency, and output the converted radio signal similarly to the first embodiment. The strength detector 225 detects the strength of the radio signal received by each of the receiving antennas A1 to An, converts an analogue signal corresponding to the strength to a digital signal, and then output the converted digital signal. The selection controller 226 determines the receiving antenna A to be used based on the digital signal which corresponds to the received strength and is output from the strength detector 225, and performs a control of instructing the selecting unit 221. In addition, the selection controller 226 outputs number information which indicates the selected receiving antenna among the receiving antennas A1 to An, and strength information which indicates the received strength of each of the receiving antennas A1 to An. The converter 227 converts the number information and the strength information output from the selection controller 226 into a radio signal of the second frequency and outputs the converted radio signal. The insertion unit 228 inserts or superimposes the signal which corresponds to the number information and the strength information and is output from the converter 227 into the signal which is transmitted from the capsule endoscope 3 and output from the frequency converter 22, and outputs the inserted or superimposed signal. The signal input from the frequency converter 22 and the converter 227 to the insertion unit 228 is converted into a signal of the second frequency. The transmitting antenna 23 transmits the signal which is transmitted from the capsule endoscope 3 and output from the insertion unit 228, and the signal corresponding to the number information and the strength information output from the selection controller 226 at the second frequency.

On the other hand, the external unit 202b includes a signal processor 233 instead of the signal processor 33 according to the first embodiment. The signal processor 233 processes the signal output from the receiver 32 via the receiving antenna 31 and extracts image information and the like transmitted from the capsule endoscope 3, similarly to the first embodiment. The signal processor 233 further extracts the number information and the strength information output from the selection controller 226, detects a position of the capsule endoscope 3 inside the subject 1 based on the extracted number information and strength information, and outputs the position information which indicates the detected position. The control unit 34 outputs the image information and the position information output from the signal processor 233 to the storage unit 35 and the display unit 36.

In the antenna unit 202a according to the second embodiment, the number information which indicates the receiving antenna selected as a receiving antenna for receiving a radio signal among the receiving antennas A1 to An and the strength information which indicates the received strength of each of the receiving antennas A1 to An is converted into a radio signal of the second frequency, the converted radio signal is inserted or superimposed into the radio signal transmitted from the capsule endoscope 3, and the inserted or superimposed radio signal is wirelessly transmitted to the external unit 202b.

Since the receiving system 202 according to the second embodiment is configured to wirelessly connect the antenna unit 202a and the external unit 202b and the antenna unit 202a and the external unit 202b are not connect by using a connector, a cable, or the like similarly to the first embodiment, it is possible to extend the useful life of the entirety of the receiving system 202 without using a connector whose life is generally short, and to extend the life of the entirety of the receiving system 202.

Besides, since the antenna unit 202a and the external unit 202b are not connected with a cable in the receiving system 202 according to the second embodiment similarly to the first embodiment, it is not necessary to perform a complicated work of a cable wiring for connecting the antenna unit 202a and the external unit 202b on the subject 1, and it is possible to reduce a physical strain on the patient as a subject.

Furthermore, by using a sealing member or an adhesive agent in performing a processing of attaching a battery constituting the power supply unit 24 and of assembling the frame similarly to the first embodiment, it is possible to easily secure a drop-proof property in the antenna unit 202a according to the second embodiment.

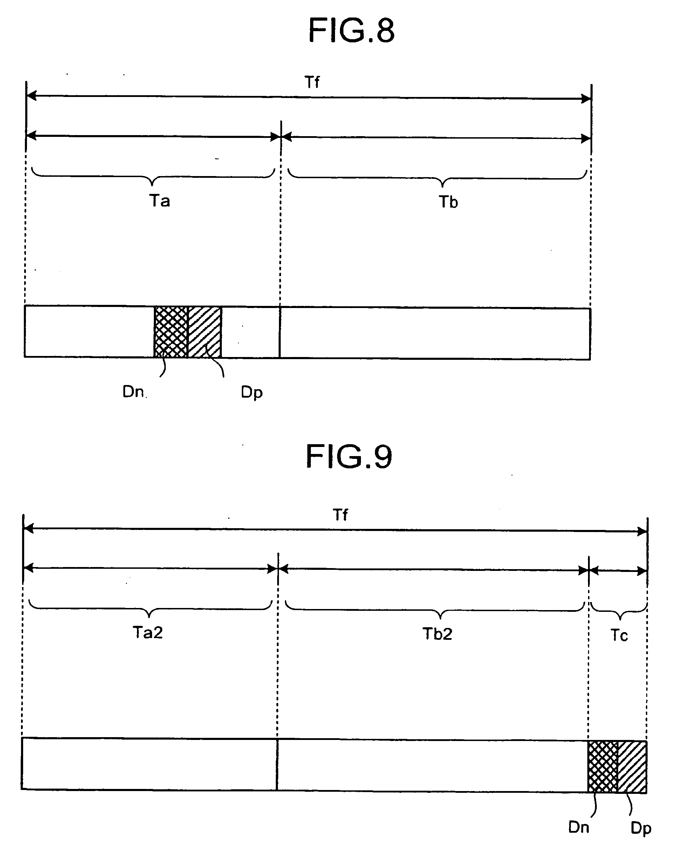

The insertion unit 228 of the antenna unit 202a may output a signal after superimposing or inserting a signal corresponding to the number information and the strength information output from the selection controller 226 on or into the radio signal transmitted from the capsule endoscope 3. For example, when the radio signal transmitted from the capsule endoscope 3 includes image information, the insertion unit 228 may superimpose a signal corresponding to number information Dn and strength information Dp on an idling period Ta which is provided before an image information period Tb including the image information within one frame period Tf corresponding to one image and includes a predetermined synchronization signal component as shown in FIG. 8. Alternatively, as shown in FIG. 9, the insertion unit 228 may have an insertion period Tc into which the signal corresponding to the number information Dn and the strength information Dp is inserted, after an image information period Tb2 present after an idling period Ta2 within the one frame period Tf. In this manner, it is only necessary that the insertion unit 228 superimposes or inserts the signal corresponding to the number information and the strength information on or into any position within the one frame period where the signal processor 233 of the external unit 202b can perform the extraction so that the position information with respect to the capsule endoscope 3 can be obtained.

The antenna units 2a and 202a may perform a conversion into a frequency of an integral multiple of the frequency of the radio signal transmitted from the capsule endoscope 3 or into a frequency which is away from the frequency of the radio signal transmitted from the capsule endoscope 3 and is not confused with the frequency of the radio signal transmitted from the capsule endoscope 3, and may transmit the converted radio signal. In this case, the external units 2b and 202b receive the radio signal transmitted from the transmitting antenna 23 of the antenna units 2a and 202a at the receiving antenna 31. Since the antenna unit 2a and the external unit 2b, and the antenna unit 202a and the external unit 202b are wirelessly connected in this case too, the receiving systems 2 and 202 have the same effect as the case of transmitting a signal at the gigahertz range frequency. Further, even in the case of arranging a device which outputs a radio signal of the gigahertz range frequency in the environment of the receiving systems 2 and 202, the radio signal transmitted from the antenna units 2a and 202a to the external units 2b and 202b never interferes with a radio wave of the gigahertz range frequency output from this device, and the antenna units 2a and 202a can accurately transmit the radio signal from the capsule endoscope 3 respectively to the external units 2b and 202b.

The antenna units 2a and 202a may first encode the radio signal from the capsule endoscope 3 and then transmit the encoded radio signal from the transmitting antenna 23. Here, the external units 2b and 202b decode the signal received via the receiving antenna 31 to acquire image information and the like. In this case, even in the case of arranging the device which receives a signal of the same frequency range as the radio signal transmitted from the antenna units 2a and 202a in the environment of the receiving systems 2 and 202, the radio signal transmitted from the antenna units 2a and 202a is not processed by the device, and a confusion with processing information can be prevented in the receiving systems 2 and 202 and in their peripheral equipment.

Though each of the antenna units 2a and 202a described above is configured to have the power supply unit 24, the present invention is not limited to this configuration and the power may be wirelessly supplied from the external units 2b and 202b.

### INDUSTRIAL APPLICABILITY

As described above, the antenna unit and the receiving system according to the present invention are useful as an antenna unit which receives a radio signal transmitted from a body-insertable device and a receiving system, and specifically suitable for a case where a reduction of a physical strain on a patient as a subject is desired.

## Claims

1. A receiving system (2; 202), comprising:
an antenna unit (2a; 202a) including
a first antenna (A1; A1 to An) which is adapted to be arranged on an outer surface of a subject (1) and receives a radio signal transmitted at a first frequency from a body-insertable device (3) inserted into an inside of the subject (1),
a main body part which is adapted to be attached to an outside of the subject (1) and includes a frequency converter (22) that converts the signal of the first frequency received by the first receiving antenna (A1; A1 to An) into a signal of a second frequency and a transmitting antenna (23) that transmits the signal of the second frequency as a radio signal, and
a signal line (21a) which connects the first receiving antenna (A1; A1 to An) and the main body part; and
an external unit (2b; 202b) including
a second receiving antenna (31) which receives the radio signal of the second frequency transmitted from the transmitting antenna (23), and
a reception processor (32) which demodulates the radio signal received by the second receiving antenna (31) to acquire information transmitted from the body-insertable device (3);
wherein the first receiving antenna (A1; A1 to An) and the signal line (21a) are provided in plural number, and
the main body part includes
a received-strength detector (225) that detects a received strength of the radio signal received by each of the first receiving antennas (A1; A1 to An),
a selection controller (226) that selects one first receiving antenna for receiving the radio signal based on the received strength detected by the received-strength detector (225),
a switching unit (221) that switches over to the first receiving antenna, selected by the selection controller (226), among the plurality of first receiving antennas (A1; A1 to An), and
a converting unit (227, 228)
that converts selected antenna identifying information for the first receiving antenna selected by the selection controller (226) into a radio signal of the second frequency, and performs one of inserting into or superimposing the converted radio signal on the radio signal which is transmitted from the body-insertable device (3) and converted by the frequency converter (22), or
that converts, into a radio signal of the second frequency, antenna identifying information for identifying each of the first receiving antennas (A1; A1 to An) and the received-strength of each radio signal received by each of the first receiving antennas (A1; A1 to An) which are detected by the received-strength detector 225), and performs one of inserting into or superimposing the converted radio signal on the radio signal which is transmitted from the body-insertable device (3) and converted by the frequency converter (22).

## Patentansprüche

1. Empfangssystem (2; 202), mit:
einer Antenneneinheit (2a; 202a), aufweisend:
eine erste Antenne (A1; A1 bis An), die so eingerichtet ist, dass sie an einer Außenoberfläche einer Person (1) angeordnet werden kann und ein Funksignal mit einer ersten Frequenz von einem in den Körper einführbaren Gerät (3) empfängt, das in das Innere der Person (1) eingeführt ist,
einem Hauptkörperteil, der so eingerichtet ist, dass er an einer Außenseite der Person (1) angeordnet werden kann und einen Frequenzwandler (22) enthält, der das Signal mit der ersten Frequenz, das von der ersten Empfangsantenne (A1; A1 bis An) empfangen wird, in ein Signal mit einer zweiten Frequenz wandelt, und einr Sendeantenne (23), die das Signal mit der zweiten Frequenz als ein Funksignal sendet, und
einer Signalleitung (21a) die die erste Empfangsantenne (A1; A1 bis An) und den Hauptkörperteil verbindet; und
einer externen Einheit (2c; 202b), mit
einer zweiten Empfangsantenne (31), die das Funksignal mit der zweiten Frequenz, das von der Sendeantenne (23) gesendet wird, empfängt, und
einem Empfangsprozessor (32), der das von der zweiten Empfangsantenne (31) empfangene Funksignal demoduliert, um Informationen zu erfassen, die von dem in den Körper einführbaren Gerät (3) gesendet werden;
wobei eine Mehrzahl erste Empfangsantennen (A1; A1 bis An) und Signalleitungen (21a) bereitgestellt ist, und
der Hauptkörperteil enthält:
einen Empfangsstärkedetektor (225), der die Empfangsstärke des von den ersten Empfangsantennen (A1, A1 bis An) empfangenen Funksignals erfasst,
eine Wahlsteuerung (226), die eine erste Empfangsantenne zum Empfangen des Funksignals auf Basis der vom Empfangsstärkedetektor (225) erfassten Empfangsstärke wählt,
eine Schalteinheit (221), die auf die aus der Mehrzahl erster Empfangsantennen von der Wahlsteuerung (226) gewählte erste Empfangsantenne (A1, A1 bis An) umschaltet, und
eine Wandlereinheit (227, 228),
die die gewählte Antennenidentifizierungsinformation zum Identifizieren der von der Wahlsteuerung (226) gewählten ersten Empfangsantenne in ein Funksignal mit der zweiten Frequenz wandelt und entweder das Einfügen in oder das Überlagern des gewandelten Funksignals über das Funksignal ausführt, das von dem in den Körper einführbaren Gerät (3) gesendet und vom Frequenzwandler (22) gewandelt wird, oder
die die Antennenidentifizierungsinformation zum Identifizieren jeder der ersten Empfangsantennen (A1, A1 bis An) und die Empfangsstärke, die vom Empfangsstärkedetektor (225) erfasst wird, jedes von jeder der ersten Empfangsantennen (A1, A1 bis An) empfangenen Funksignals in ein Funksignal mit der zweiten Frequenz wandelt und entweder das Einfügen in oder das Überlagern des gewandelten Funksignals über das Funksignal ausführt, das von dem in den Körper einführbaren Gerät (3) gesendet und vom Frequenzwandler (22) gewandelt wird.

## Revendications

1. Système de réception (2 ; 202), comprenant :
une unité d'antenne (2a ; 202a) incluant
une première antenne (A1 ; A1 à An) qui est adaptée pour être disposée sur une surface externe d'un sujet (1) et reçoit un signal radio transmis à une première fréquence depuis un dispositif pouvant être inséré dans un corps (3) inséré à l'intérieur du sujet (1),
une partie de corps principal qui est adaptée pour être fixée à l'extérieur du sujet (1) et comprend un convertisseur de fréquence (22) qui convertit le signal de la première fréquence reçu par la première antenne de réception (A1 ; A1 à An) en un signal d'une deuxième fréquence et une antenne de transmission (23) qui transmet le signal de la deuxième fréquence en tant que signal radio, et
une ligne de signal (21a) qui connecte la première antenne de réception (A1 ; A1 à An) et la partie de corps principal ; et
une unité externe (2b ; 202b) incluant
une deuxième antenne de réception (31) qui reçoit le signal radio de la deuxième fréquence transmis depuis l'antenne de transmission (23), et
un processeur de réception (32) qui démodule le signal radio reçu par la deuxième antenne de réception (31) pour obtenir des informations transmises depuis le dispositif pouvant être inséré dans un corps (3) ;
dans lequel la première antenne de réception (A1 ; A1 à An) et la ligne de signal (21a) sont prévues en plusieurs exemplaires, et
la partie de corps principal comprend
un détecteur d'intensité de réception (225) qui détecte une intensité de réception du signal radio reçu par chacune des premières antennes de réception (A1 ; A1 à An),
un contrôleur de sélection (226) qui sélectionne une première antenne de réception pour recevoir le signal radio sur la base de l'intensité de réception détectée par le détecteur d'intensité de réception (225),
une unité de commutation (221) qui commute vers la première antenne de réception, sélectionnée par le contrôleur de sélection (226), parmi la pluralité de premières antennes de réception (A1 ; A1 à An), et
une unité de conversion (227, 228)
qui convertit les informations d'identification d'antenne sélectionnée destinées à identifier la première antenne de réception sélectionnée par le contrôleur de sélection (226) en un signal radio de la deuxième fréquence, et réalise l'une parmi l'insertion ou la superposition du signal radio converti sur le signal radio qui est transmis depuis le dispositif pouvant être inséré dans un corps (3) et converti par le convertisseur de fréquence (22), ou
qui convertit, en un signal radio de la deuxième fréquence, les informations d'identification d'antenne destinées à identifier chacune des premières antennes de réception (A1 ; A1 à An) et l'intensité de réception de chaque signal radio reçu par chacune des premières antennes de réception (A1 ; A1 à An) qui est détectée par le détecteur d'intensité de réception (225), et réalise l'une parmi l'insertion ou la superposition du signal radio converti sur le signal radio qui est transmis depuis le dispositif pouvant être inséré dans un corps (3) et converti par le convertisseur de fréquence (22).
